# EUROPEAN PATENT SPECIFICATION

(11) **EP 3 027 693 B1**
(45) Date of publication and mention of the grant of the patent: **02.07.2025**
(21) Application number: 14758692.9
(22) Date of filing: 01.08.2014
(51) Int. Cl.: C09D 5/00

(54) **A COATING COMPOSITION COMPRISING A DYE AND A METHOD TO DETECT MOISTURE IN OBJECTS**
BESCHICHTUNGSZUSAMMENSETZUNG MIT EINEM FARBSTOFF UND VERFAHREN ZUR DETEKTION DER FEUCHTIGKEIT IN OBJEKTEN
COMPOSITION DE REVÊTEMENT COMPRENANT UN COLORANT ET PROCÉDÉ POUR DÉTECTER L'HUMIDITÉ DANS DES OBJETS

(30) Priority: 02.08.2013 EP 13179121
(43) Date of publication of application: 08.06.2016
(73) Proprietor: Nederlandse Organisatie voor toegepast- natuurwetenschappelijk onderzoek TNO, 2595 DA 's-Gravenhage (NL)
(72) Inventor: MEULENDIJKS-KIGGEN, Nicole Maria Matthias, 2595 DA 's-Gravenhage (NL); ERICH, Sebastiaan Joannes Franciscus, 2595 DA 's-Gravenhage (NL); EVERSDIJK, Jacobus, 2595 DA 's-Gravenhage (NL); PAPEN-BOTTERHUIS, Nicole Ellen, 2595 DA 's-Gravenhage (NL); SALARI, Johannes Wilhelmus Otto, 2595 DA 's-Gravenhage (NL)
(74) Representative: V.O.
(86) International application number: PCT/NL2014/050541
(87) International publication number: WO 2015/016716

(56) References cited:
- WO-A1-2013/037928
- WO-A2-03/070138
- US-A- 2 254 609
- US-A- 4 990 284
- US-A- 5 435 010
- US-A- 5 440 927
- M. KLEEMANN ET AL: "POLYMER FILM DOPED WITH A SOLVATOCHROMIC DYE FOR HUMIDITY MEASUREMENT", PROCEEDINGS OF SPIE, OPTICAL MATERIALS AND APPLICATIONS, vol. 5946, 12 June 2006 (2006-06-12), BELLINGHAM, pages 1 - 7, XP002722658, DOI: 10.1117/12.639189
- BOSCH P ET AL: "Polyurethane-acrylate based films as humidity sensors", POLYMER, ELSEVIER SCIENCE PUBLISHERS B.V, GB, vol. 46, no. 26, 12 December 2005 (2005-12-12), pages 12200 - 12209, XP027728356, ISSN: 0032-3861, [retrieved on 20051212]

## Description

The invention relates to the detection of moisture in coated objects. In particular, the invention relates to a coating composition comprising a dye and to a method to detect moisture in objects using a signalling layer comprising the coating composition. Such coatings, for example, can be useful in detecting moisture build-up on wood or metal surfaces.

In the broad sense, the invention relates to the area of responsive coatings. Such coatings, according to a general definition, are able to detect changes in the environment, e.g. in the underlying surface, and make these changes "visible" through the signalling function. The signalling function can be very diverse in nature but preferably it allows to "read out" the changes at a distance. The signalling function can for example be the change of absorption properties of the coating (e.g., colour).

However, it is undesired for the coatings used for decorative purposes such as paints for wood or other surfaces to change colour under the influence of the environment. Therefore, responsive coatings should preferably not absorb in the visible spectrum, i.e. have no colour. (Near) Infrared (IR) and ultraviolet (UV) dyes, that is, dyes that absorb in the (near) infrared area or ultraviolet area, respectively, meet this requirement and are particularly suitable in coatings with a signalling function. In principle, the solar spectrum can be used as a light source enabling the signalling function.

Coatings comprising microparticles with NIR dyes are for example known from US 8,367,193. This patent describes nanocomposite dispersions which are used to provide transparent coatings having enhanced barrier properties featuring an invisible marker dye in the dispersion for improved quality control, security, and manufacturing development. The marker dye is a water soluble infrared (IR) or near infrared (NIR) dye. The coatings provide transparent high barrier coatings at thicknesses from 1-10 microns that include an IR or an NIR dye that enables rapid measurement of coating thickness and uniformity. The described coating is however unsuitable to detect any changes in the environment as the described polymer-dye system is stable and not susceptible to a change. Thus, the described coating is not a responsive coating.

One of the important functionalities of responsive coatings is the detection of moisture build-up in the surfaces under the coatings, such as wooden surfaces, metal surfaces or other surfaces.

For wooden surfaces, existing methods to detect moisture are mainly destructive, e.g. wherein two pins are inserted in a wooden object to measure the moisture resistance in the wood. Non-destructive methods based on e.g. unilateral mobile NMR and infrared thermography are not always sufficiently precise or applicable. Also, for other types of surfaces non-destructive methods are not known.

Moreover, existing methods only allow a local measurement of the moisture content of the surface under the coating, and not of the whole surface at once.

WO 2013/037928 describes a coating formed on a substrate, wherein the coating comprises (a) an organic NIR-transparent pigment and/or an inorganic NIR-reflective pigment; (b) and a dye having a transmittance of at least 75% in the range of from 700 to 2500 nm.

M. Kleemann et al, "POLYMER FILM DOPED WITH A SOLVATOCHROMIC DYE FOR HUMIDITY MEASUREMENT", PROCEEDINGS OF SPIE, OPTICAL MATERIALS AND APPLICATIONS, BELLINGHAM, (20060612), vol. 5946, doi:10.1117/12.639189, XP002722658, describes a polymer film doped with a solvatochromic dye for humidity measurement.

Bosch P et al, "Polyurethane-acrylate based films as humidity sensors", POLYMER, ELSEVIER SCIENCE PUBLISHERS B.V, GB, vol. 46, no. 26, ISSN 0032-3861, (20051212), pages 12200 - 12209, XP027728356, describes polyurethane-acrylate based films as humidity sensors.

US4990284 describes a solution-type moisture indicating ink to be printed on a substrate and adapted to change color at a preselected ambient moisture.

WO 03/070138 describes a wetness indicating composition comprising a colorant disposed in a carrier matrix.

US5440927 describes a fiber optic moisture sensor. A film comprising an optically transparent polymer and a salt complex of a metal ion and an organic compound is used.

US2254609 describes a paint-like composition which, wen set, indicates liquid leakage.

US5435010 describes an article of clothing that changes color when exposed to varying moisture levels within the article.

An object of the present invention is to provide a coating for different kinds of surfaces that is suitable for detecting moisture in objects. It is a further object of the present invention to provide a method to detect moisture in objects that is non-destructive, precise and allows to analyse the whole object at once. It is also desired that the method is simple in use and does not require costly detection equipment.

In order to better address one or more of the foregoing desires, the invention, in one aspect, provides a coating composition as defined in claim 1.

The invention is based on the judicious insight that a rapid, non-destructive and simple moisture detection of large surfaces can be realised using a signalling layer with encapsulated systems comprising a dye and a polymer, wherein the dye does not absorb in the visible spectrum while showing absorption in a non-visible part of the spectrum, and wherein the encapsulated system providing the combination of the dye and the polymer is able to change absorption properties under influence of a solvent (preferably moisture) in the environment. It can also be said that the dye only shows absorption (above detection limits) in the non-visible part of the spectrum. The system can also be used to detect the presence of other solvents than moisture.

The system comprising a dye encapsulated in a matrix of a polymer is preferably realized in the form of microparticles. In a preferred embodiment, as described below in more details, the microparticles have a core-shell structure wherein the core comprises the dye and the shell comprises the polymer. This has as an advantage that the encapsulated system shows high chemical stability and is also easy to handle. In an alternative embodiment, the system is realized in the form a (semi-)continuous matrix of the polymer wherein the dye particles are dispersed.

In such a signalling coating the absorption properties of the dye can be changed, from absorbing to non-absorbing or vice versa, due to a trigger; in the invention as claimed the encapsulated system is able to undergo a detectable change of at least 10% in absorption units (AU) in the absorption properties under the influence of a solvent. The system can thus undergo a detectable change in the absorption properties at a particular wavelength. The change should be detectable with conventional means such as by a modified camera. In the invention as claimed the encapsulated system is able to undergo a detectable change of at least 10% in absorption units (AU) in the absorption properties under the influence of a solvent preferably at least 20%, more preferably at least 50%. The change of state of the dye can for example be triggered by the change of polarity by solvents, redistribution over the coating or redistribution of particle sizes of the dispersed dye.

Particularly, in one embodiment, such trigger can be pH, or the proton activity in the surface, which is in direct contact with the coating comprising the dye. The composition therefore in one embodiment comprises a pH sensitive polymer. The presence of moisture in the object near the coating at certain proton activity or pH, at which the pH sensitive polymer exhibits hydrophilic properties, leads to the rupture or disintegration of the capsules with the dye in the coating due to the swelling or hydrolysis of the polymer. Other examples of triggers are other solvents than water. In other embodiments, the polymer is pH degradable. Such polymer does not swell upon action of the trigger but degrades leading to the permeability of the polymer shell.

If the encapsulated system also contains a solvent and the dye is dissolved in that solvent, the disintegration then leads to the solvent diffusing out of the capsules and a change in the state of the dye. This can for example be the change from a solvated to a non-solvated state. The change can also be in the altered concentration of the dye, or a different dispersion of the dye in the matrix, etc.

It is also possible that a solvent is encapsulated in the polymer matrix separately from the dye, and after disintegration of the capsule, the solvent diffuses to the dye and at least partially dissolves it. In all these embodiments, the dye changes its absorption properties, for example, it does not absorb in the IR or UV area anymore. The change in absorption can be detected with a suitable camera.

A preferred embodiment of the invention is a coating comprising microparticles applied to a wooden surface and a pH as the trigger for a switch in the state of an IR dye. However, this does not limit the application of this invention, which can be applied to other surfaces, such as metal surfaces, and also other triggers such as change of polarity by solvents, etc.

In a preferred embodiment, microcapsules containing a dye dissolved in a solvent within the microcapsule shell, are uniformly distributed in a coating matrix, e.g. paint. Water from the surrounding environment comes in contact with the wood and generates acids, such as tannic acids. The resulting decrease of the pH under the coating serves as a trigger for the decomposition of the microcapsule shell. This allows the encapsulated solvent to diffuse in the coating matrix. When the dye has a very low solubility in the coating matrix it will not diffuse into the coating matrix and precipitate, resulting in a decrease of absorption signal. Alternatively, the dye is dispersed in its undissolved state in the coating matrix and the diffusion of solvent in the coating dissolves the active ingredient leading to an increase in absorption signal. The decrease (or possible increase) in absorption is the signal for the moisture getting in contact with the wood.

The system used in the coating according to the present invention comprises a dye in the matrix of a polymer. Preferably, the system is present in the form of microparticles, which preferably comprise a core and a shell surrounding the core. It is to be understood that a microparticle can also have multiple cores; likewise, also multiple shells are possible. The microparticles preferably have a particle size less than 30 µm, preferably less than 10 µm, more preferably less than 1 µm. Also other structures of the microparticle are possible, e.g. solid dye particles being dispersed, or embedded, in the polymer matrix. In that case, the solid dye particles preferably have a particle size less than 30 µm, preferably less than 10 µm, more preferably less than 1 µm. In the present context, the particle size refers to the largest particle diameter as measured on a representative number of microscope photos (such as 20).

In one embodiment, the encapsulates system further comprises a solvent. This can be a solvent in which the dye is soluble, the dye thus being present in a solvated form. It can also be a non-solvent for the dye. The solvent can also be present in the encapsulated system (microparticle) in an encapsulated form itself, so that no interaction between the solvent and the dye takes place before the polymer matrix disintegrates under the influence of the trigger. In this case the dye is present initially in a non-solvated form in the system and becomes solvated after the disintegration. The encapsulated system can also comprise a plasticizer, which can also act as a solvent for the dye. A skilled person is able to choose a suitable plasticizer for a particular system. In one embodiment, aliphatic esters are used as a plasticizer, preferably, 1,2-cyclohexane dicarboxylic acid diisononyl ester (DINCH, Hexamoll^{®}).

Particularly, the change in the dye properties under the influence of a solvent is the change in the absorption properties of the solvated and the non-solvated states. For example, one of these states should exhibit absorption in a certain (IR or UV) area, while the other state should exhibit substantially no absorption in the same area, or at least absorption in a lesser degree. Substantially no absorption means here at least 10 times lower absorption than in the absorbing state. In one such embodiment, the dye in a solvated form preferably absorbs in the IR area, while in the non-solvated form it does not absorb in that area. In another embodiment, the dye in a solvated form does not absorb in the IR area, while in the non-solvated form it absorbs in that area. Particularly, the dye in the solvated form preferably absorbs light with a wavelength in the range of 700-2400 nm, and in a non-solvated form it does not absorb light in that area, or vice versa.

Preferably, the dye is an IR or UV dye, which means that the dye shows absorption in, respectively, the IR or the UV part of the light spectrum. In the invention defined in claim 1, the dye only shows absorption in the non-visible part of the light spectrum. In some embodiments, the dye is an IR dye.

It is preferred that the dye exhibits a single strong absorption peak in the IR or UV area. Preferably, in the most absorbing state (e.g. solvated form), the dye has an absorption peak in the wavelength region 700-2400 nm or 10-400 nm, more preferably 750-1200 nm. In the other state (e.g. non-solvated form), the dye should not absorb light in the same area, or absorb in a lesser degree. Preferably, in the less absorbing state (e.g. non-solvated) the dye should absorb less than 50%, preferably less than 30%, more preferably less than 10% than the most absorbing state (e.g. solvated), measured as the ratio of intensities at a same wavelength. The same applies to the case when a non-solvated form absorbs the most in the non-visible light spectrum (IR or UV area).

The solvated state refers to the situation in which the dye can molecularly interact with the matrix and/or a solvent present, in such a manner that it will change the absorption behaviour of the dye molecule relative to the non-solvated state. The non-solvated state refers to the situation in which the majority of the dye molecules do not interact with the matrix and/or a solvent.

Without wishing to be bound by particular theory, it is believed that in order to have optical effects, the distribution of the particles in the coating is relevant, since only the surface molecules of a finely dispersed powder have optical effects. It is also believed that only these surface molecules interacting with the matrix and/or solvent provide the optical effects and not the bulk. Since the dyes have such molecular interaction, the behaviour can be switched on and off by changing the compatibility with the environment, e.g. use of a non-solvent or solvent. This allows the dyes to change from an absorbing to a non-absorbing state and vice-versa.

The dye used in the present invention essentially does not absorb light in the visible region (wavelength in the range 400-700 nm) and therefore has no colour for the naked eye. The non-absorption in the visible spectrum allows the dye to be used in decorative coatings without affecting the colour of the coating (e.g. paints).

Suitable dyes can for example be organic dyes, polymeric dyes, metal complexes, plasmonic particles. Some specific examples of particularly suitable NIR dyes are NIR1002 and NIR1047 of QCR. Examples of UV dyes include coumarin and its derivatives, such as 7-hydroxy-4-methylcoumarin or 7-hydroxy-3-methylcoumarin, but also benzophenones, p-aminobenzoic acid, camphor, dibenzoylmethane, homosalate, cinnamates, crylenes and derivatives thereof. In a particular embodiment, non-fluorescent dyes are used. A skilled person is able to determine which dyes do not show fluorescence. Fluorescence in the visible spectrum is even undesirable, e.g. in decorative coatings it would influence the colour of the coating (paint).

A group of preferred organic dyes includes quaterrylenes, quinophthalones, anthraquinones, dioxazines, cyanines. Suitable dyes are for example LUMOGEN^{®} from BASF like LUMOGEN 765, a quaterrylene dye with an absorption maximum at 765 nm, NIR dyes from QCR Solutions, ADS and FEW Chemicals.

Some suitable NIR absorbing organic dyes are shown in Table 1 herein-below together with their chemical formula (if known) and the absorption maximum wavelength.

**Table 1**

| Compound | Chemical structure | λₘₐₓ |
|---|---|---|
| Lumogen IR765 | 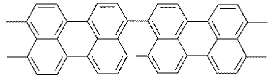 | 765 nm |
| NIR886A | | 886 nm |
| NIR 1002A | | 1002 nm |
| NIR1047A | | 1047 nm |
| ADS830WS | 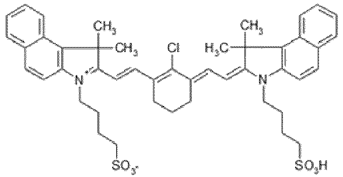 C₅₂H₆₆ClN₃O₆S₂ | 830 nm |
| S2161 | 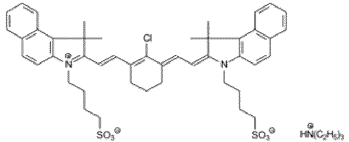 C₅₂H₆₆ClN₃O₆S₂ | 820 nm |
| S0837 | 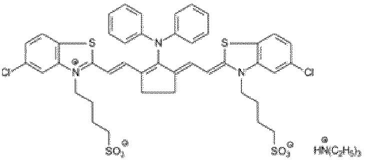 C₄₉H₅₆Cl₂N₄O₆S₄ | 808 nm |
| Lumiprobe Cy7.5 | 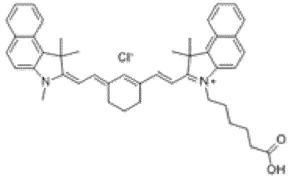 C₄₅H₄₉ClN₂O₂ | 788 nm |

Another group of suitable dyes is metal chelates, preferably nickel chelates. Suitable dyes from this group include ADS845MC, ADS920MC, ADS870MC (from ADS dyes), bis(dithiobenzyl)nickel, bis(4,4'-dimethoxydithiobenzyl) nickel, bis(4-dimethylaminodithiobenzyl)nickel. These dyes are shown in Table 2 below with their chemical structure and the absorption maximum wavelength.

**Table 2**

| Compound | Chemical structure | λₘₐₓ |
|---|---|---|
| ADS845MC | 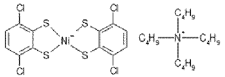 | 845 nm |
| ADS920MC | 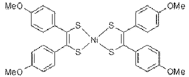 | 920 nm |
| ADS870MC | 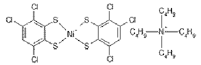 | 870 nm |
| Bis(dithiobenzyl) nickel | 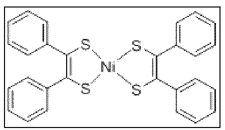 | 866 nm |
| Bis(4,4'-dimethoxy dithiobenzyl) nickel | 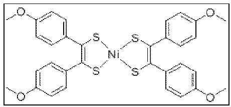 | 925 nm |
| Bis(4-dimethylamino dithiobenzyl)nickel | 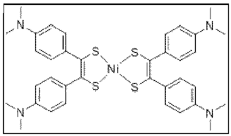 | |

In a preferred embodiment, the encapsulated system further comprises a solvent. The dye can be dissolved in the solvent, or it can be encapsulated separately to prevent the contact. The dye can thus be present in a solvated form, or in a non-solvated form initially in the microparticle. Any suitable solvent wherein the dye is soluble, can be used. In some embodiment, solvents wherein the dye is not soluble are used, e.g. in combination with other solvents wherein the dye is soluble - in this way the solubility of the dye can be influenced. A skilled person is able to find a suitable solvent for a particular dye.

In some embodiments, the solvent is preferably not water and the solution is thus non-aqueous. Preferably, an organic solvent is used. Accordingly, in these embodiments the dye is not a water-soluble dye. Typically, organic solvents such as ketones, esters, and alcohols are suitable. For example, for the NIR1002 and NIR1047 dyes a particularly suitable solvent is methyl isobutyl ketone (MIBK).

The dye is preferably present in the solution in an amount of less than 1 wt.%, preferably less than 0.1 wt.%, more preferably at most 0.01 wt.%, such as 0.00001 - 0.01 wt.%, preferably 0.0001-0.005 wt.%.

The composition according to the invention comprises further a polymer. In one embodiment, the polymer is a pH sensitive polymer and preferably have a critical swelling pH under 7, more preferably at 2.5-6.0, yet more preferably 2.6-5.8, most preferably 3.0-5.0. This pH range is particularly useful for wood surfaces, since it has been found by the inventors to correspond to the pH in the wood which has been subjected to moisture. This pH is applicable to all non-chemically treated wood sorts. Preferably, the wood sorts include larch, fir, pine, Douglas fir, maple, birch, beech, ash, meranti, teak. In other applications, a basic critical swelling pH (that is, greater than 7, preferably at least 8) may be desirable.

The critical swelling pH of a polymer is a pH at which a switch in hydrophobic/hydrophilic properties takes place. Particularly, in one embodiment, the polymer has hydrophilic properties at a pH below the swelling pH and exists in a swelled form with hydrated polymer chains. At a pH above the critical swelling pH, it has hydrophobic properties and is in a collapsed form.Preferably, copolymers (or hydrogels) having moieties capable of being protonated and thereby becoming cationic (cationic copolymers) are used as pH-sensitive polymers in the present invention. Such copolymers contain cationic (positively charged) moieties on their backbones, that is moieties that are capable of being protonated. The copolymer shows either hydrophilic or hydrophobic properties depending on the pH of the environment. At pH values higher than the pKa of the cationic moieties, the copolymer is hydrophobic (excludes water from the system) and is in the collapsed state whereas at pH values lower than the pKa the copolymer becomes hydrophilic and absorbs water resulting in a swollen state. The pH of the switch in the hydrophobic-hydrophilic properties can be referred to as the critical swelling pH. At this pH, the transition between the swollen and the collapsed states takes place.

Copolymers capable of being protonated can be prepared by the copolymerisation of monomers capable of being protonated onto a polymer backbone. Such copolymer is therefore preferably a polymer copolymerised with at least one monomer capable of being protonated. The monomer capable of being protonated is preferably selected from the group consisting of acrylates, methacrylates, vinylics containing at least one primary, secondary or tertiary amine or sulfide group and derivatives thereof.

Preferably, the polymer backbone contains at least one tertiary amine group, since these protonate at a low pH. When the pH is decreased below the pKa of the ionisable groups, protonation of the amine groups occurs resulting in the formation of positive charges along the backbone chain.

Suitable copolymers can be prepared from dimethylaminoethyl methacrylate (DMAEM) or diethylaminoethyl methacrylate (DEAEM) with methyl methacrylate (MMA). Another example is a copolymer of dimethylaminoethyl methacrylate, butyl methacrylate, and methyl methacrylate. A suitable polymer is for example Eudragit E PO.

In another embodiment, hydrolysable polyesters are used as a pH-sensitive polymer. Examples of these are polylactic acid (PLA), polyglycolic acid (PGA), poly(lactic-co-glycolic acid) (PLGA), polycaprolactones. In this embodiment, the polymer is a pH degradable polymer, which degrades at a particular pH. Preferably, the critical point is pH under 7, more preferably at 2.5-6.0, yet more preferably 2.6-5.8, most preferably 3.0-5.0. Particularly suitable pH degradable polymers are acid-catalyzed hydrolysable polyesters, preferably aliphatic-aromatic polyesters such as Ecoflex^{®} from BASF.

The encapsulated system can further contain other components, such as at least one of surfactants, dispersion aids, wetting agents, thickeners, anti-foaming agents, stabilizers.

In one embodiment, the coated surface is a wooden surface. Preferably, the wood sorts include larch, fir, pine, Douglas fir, maple, birch, beech, ash, meranti, teak.

The encapsulated system such as microparticles described above can be prepared by a method, comprising encapsulation of the dye in a matrix comprising the polymer. Any suitable encapsulation method known to the skilled person can be used, for example solvent-evaporation technique. During encapsulation surfactants can be used to reduce agglomeration, if necessary. Suitable surfactants are known to a skilled person. When the microparticles also comprises a solvent, in which the dye is dissolved, the dye may be dissolved in this solvent prior to the encapsulation.

The encapsulated system used in the invention is preferably used in the form of a dispersion of microparticles in a suitable organic or inorganic solvent, e.g. in coatings for wooden or other surfaces. The coating composition can further comprise conventional components such as a liquid phase (organic solvents or aqueous), binders, etc.

The coating composition according to the invention is generally applied to a substrate (e.g. a wooden or metal surface) and dried to form a coating. After the coating is applied, the moisture content of the coated surface can be detected due to the presence, change or lack of the absorption of the dye present in the coating.

In a further aspect, the invention provides a method to detect moisture in objects, the method comprising the steps of:
(a) providing an object with a signalling layer, said signalling comprising the coating composition according to claim 1, and
(b) measuring the light absorption or reflectance of the coated object in the non-visible part of the light spectrum.

In another preferred embodiment, the step of measuring the light reflectance or absorption is carried out at least twice, the method further comprising the step of detecting differences in the light reflectance or absorption of the object in the non-visible part of the light spectrum.

The differences can be determined in various ways. For example, this can be done in time, wherein at least two measurements of the light reflectance or absorption are carried out with a time interval, such as, weeks, months, years. In this case only the non-visible part of the spectrum should be analysed.

In another embodiment, the differences of the whole spectrum are detected, e.g. it is possible to make measurements at the same moment (although not strictly necessary) of the whole spectrum and of the non-visible part. This can also be combined with measurements with time intervals, e.g. to perform a double check, to monitor the differences in the visible and non-visible part of the spectrum in time. In this embodiment also image analysis can be used, for example if a visibly smooth coating has an irregular pattern in UV or IR, the presence of irregularities (spots) may point to the spots with moisture present. This makes it possible to also determine the exact place of the moisture build-up.

Preferably, in the described method the dye changes its state from absorbing to non-absorbing or vice versa, under the influence of presence of moisture.

In the present invention, the moisture is detected by detecting the reflectance or absorption (both influenced by absorption properties of the materials) of light having a wavelength in the non-visible part (IR or UV area) of the light spectrum of the coated surface. The precise wavelength range where the absorption is to be detected preferably corresponds to the absorption peak of the used dye.

In a preferred embodiment, the reflectance in the IR area can suitably be detected using a CCD camera. Current CCD chip technologies are used in everyday mirror-reflex photo cameras. The CCD chips used in these cameras have a sensitive region all the way up to 1200 nm in the infrared (IR) region. However, when taking images usually only the information from the visible spectrum is relevant and therefore filters for IR cut-off are placed in these cameras to filter out the information from the IR region. However, by removing these filters and applying alternative filters specific signals in the infra-red can be detected.

For example, by taking two images - one of the whole spectrum (IR filter switched on, or present) and one only of the visible part (IR filter switched off, or removed), image analysis can be used to only analyse the IR part of the image. In this way the region in which the absorption or reflectance has changed considerably can be identified. Specially designed software can be used for the image analysis, which is subsequently used for "assessing" the need for intervention such as maintenance, e.g. to prevent further damage.

Therefore, in a preferred embodiment, the detection is carried out by making at least two images of the coated object, more preferably a first image using a filter and a second image without using the filter, the first and the second image being made at substantially the same point in time or at substantially different points in time.

As an example, a coating can be used wherein a dye is dissolved in a solvent and both are encapsulated in a pH-sensitive polymer matrix. In case the dye is used that shows absorption in a solvated state and no absorption in a non-solvated state, the object without moisture will show absorption (e.g. dark or black areas) in the NIR picture. If the object however contains moisture, this will be visible in the NIR picture as lack of absorption.

In a preferred embodiment, the method is applied to a coated surface of an object which is a wooden or metal surface, and is preferably a wooden surface. The wood sort is preferably selected from larch, fir, pine, Douglas fir, maple, birch, beech, ash, meranti, or teak.

By detecting the absorption or reflectance of the coating at different points of time, the presence of moisture in the material underlying the coating, e.g. wood, can be monitored in time in all weather conditions. Preferably, the method is applied to objects with wooden and metal surfaces, however other applications are also possible.

The invention will now be illustrated in the following, nonlimiting examples. Parts and percentages mentioned in the examples and through the description, are by weight, unless otherwise indicated.

### Example 1: Solvent based triggering

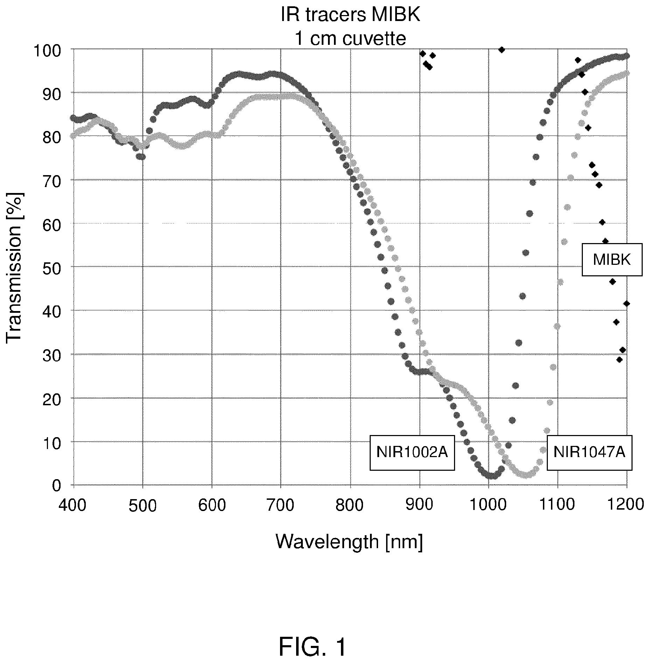

The organic NIR dye NIR1002A was chosen as signalling agent because of its high absorption coefficient in the NIR area. The transmission spectra of the dye in a dissolved state, 0.001wt.% in MIBK, measured in 1 cm cuvette, is shown in Figure 1. Figure 1 also contains the transmission spectrum of the solvent MIBK.

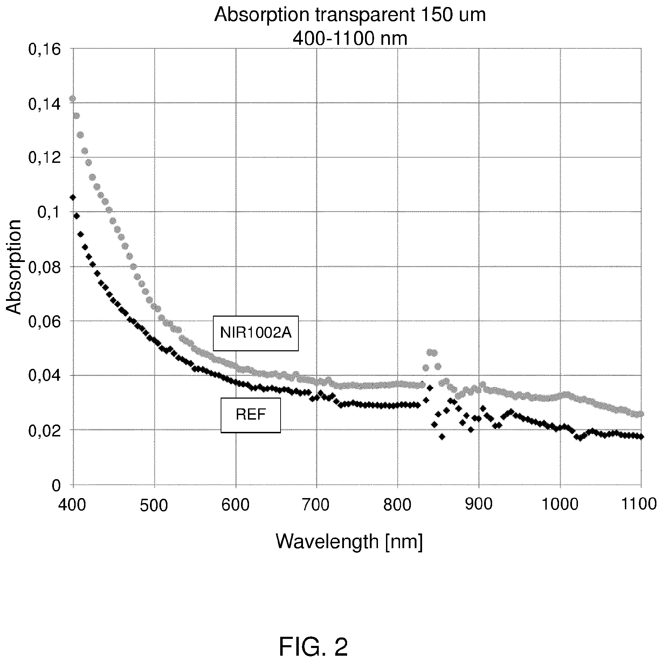

The dye is dispersed as solid particles in a polyacrylate transparent coating prepared from polyacrylate latex dispersion, in the form of coating films with the thickness of 15 microns. Figure 2 shows the absorption spectrum of the coating film with NIR1002A (upper curve) and without the dye (lower curve), in the area 400-1100 nm.

As seen in Figure 2, no absorption is measured in the NIR area of the coating films, when the dye is not solvated.

The dye NIR 1002A was formulated in the polyacrylate coating, applied on a glass substrate, and dried. To trigger the switch in the absorption properties, the lower half of the surface of the coating is wetted by the solvent MIBK. This leads to a dissolved state of the dye and switching from a non-absorbing to an absorbing state.

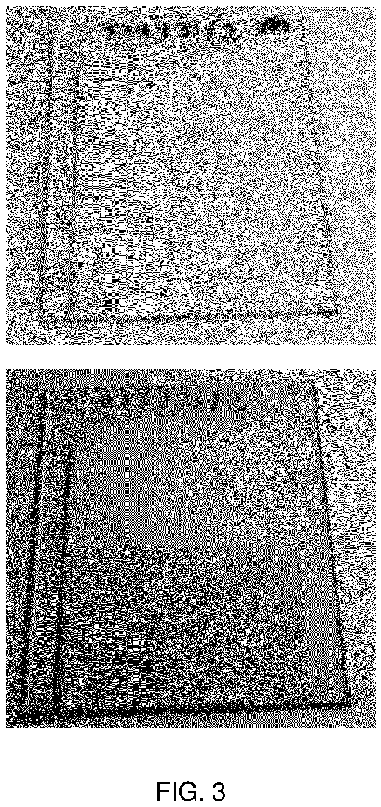

Figure 3 shows a picture taken in both the visible (top) and NIR region (bottom) using a camera. For the NIR image a filter with the cut-off 850 nm was used. Over a distance of circa 2 meter, the solvent-wetted area is clearly visible. In the visible light however, no colour change is visible. This also shows that only a low concentration of the dye is already sufficient to create the switch effect.

### Example 2: Encapsulation of NIR dye using pH degradable polymer

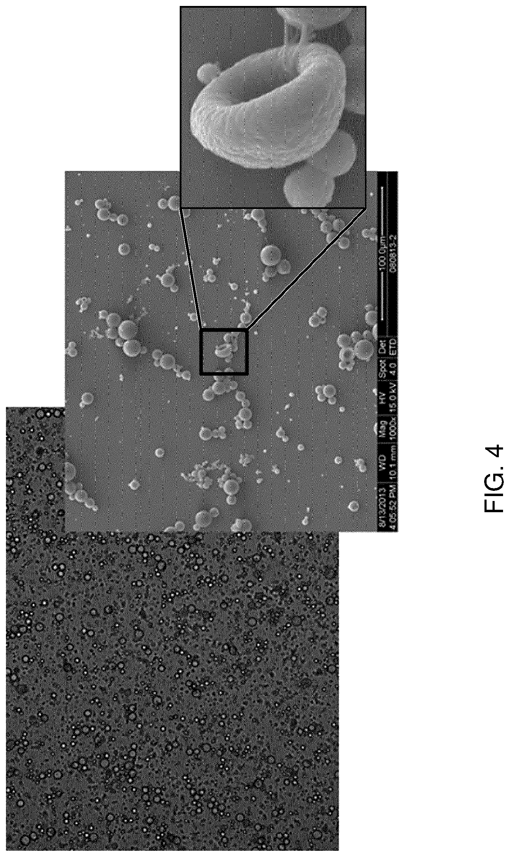

The hydrophobic NIR dye, dissolved in a hydrophobic plasticizer Hexamoll, was encapsulated with an acid-catalyzed hydrolysable polyester (Ecoflex^{®}) using solvent-evaporation technique, which results in core/shell microcapsules. During encapsulation a surfactant TAMOL^{®} (condensated naphtalenesulphonic acid)/PVA has been used to reduce aggregation. The obtained microcapsules are shown in the microscopic (SEM) images in Figure 4.

The images in Figure 4 clearly show the formation of core/shell capsules. The inset of the SEM image showing an indented capsule indicates that the formed particles are hollow. The reason for the indentation is that the environment of the SEM is a high vacuum. Even though the plasticizer has a high boiling point, at such low pressures the plasticizer evaporates. The evaporating core causes the polymer shell to collapse in itself.

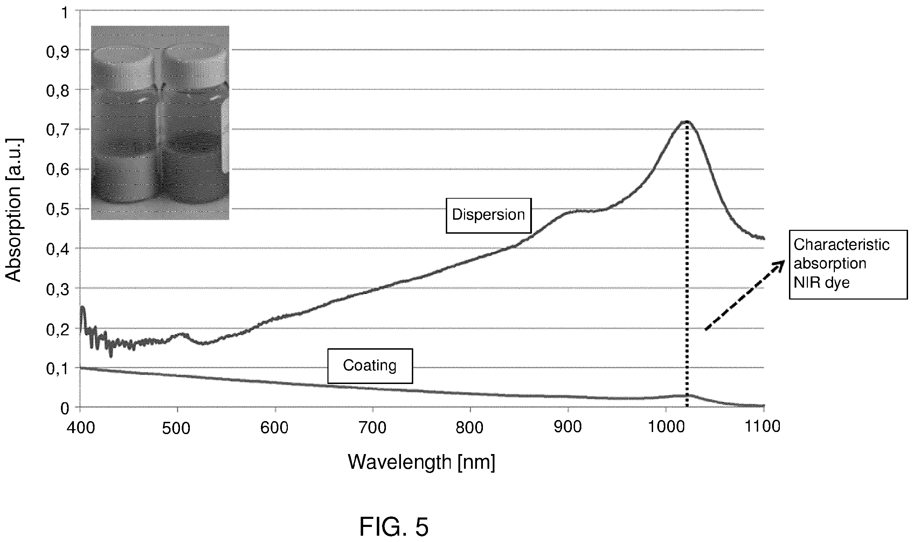

The obtained microcapsules are then incorporated in the coating. The absorption characteristics of the coating are measured with a spectrophotometer and the resulting spectra are shown in Figure 5. The lower curve corresponds to the coating with incorporated microcapsules, the upper curve corresponds to the aqueous dispersion of the microcapsules.

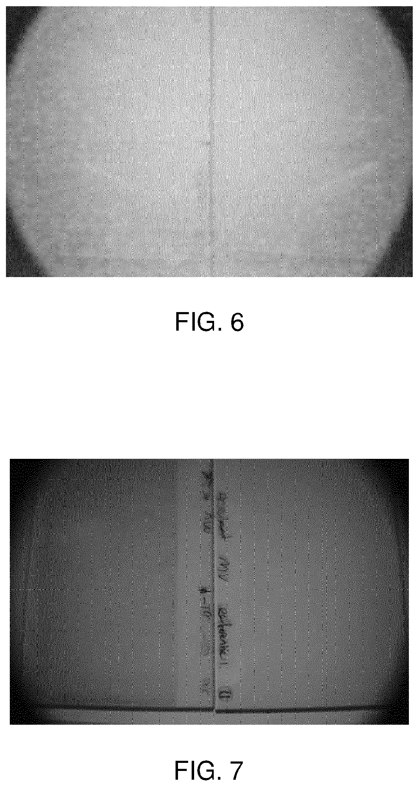

The aqueous dispersion is measured in a cuvette, while the coating is applied on a glass microscope slide and inserted as such in the spectrophotometer. The absorption was also measured with the respect to capsules containing no NIR dye and it was concluded that the absorption in Figure 6 is due to the presence of the NIR dye.

Figure 5 shows that the characteristic absorption of the NIR dye is present in both the coating and the dispersion, although to a different extent. The difference in absorption of the NIR dye in the coating and in the aqueous dispersion is explained by the thickness of the measured layer - the coating is only 75 µm thick, while the cuvet has an internal width of 5 mm. This is an order of magnitude difference which explains the large difference in absorption.

### Example 3: Particulate NIR dyes in coating systems

Moisture signalling can also be achieved by gradual dissolution of the NIR dye solid particles and redistribution of the dye within the coating. Upon dissolution and redistribution, the coating changes its optical properties from respectively non-absorbing to absorbing.

A solvent-borne alkyd coating containing a water-soluble NIR dye S2161 (FEW Chemicals) was prepared. In Figure 6, an optical image taken with a camera with a NIR filter (cut-off: 652 nm) is shown. The image shows two coatings: on the left half is the coating with the NIR dye and on the right half is the coating without the NIR dye. The figure shows that there is no difference in absorption. Both the NIR dye containing coating as well as the reference coating show no absorption in NIR spectral region.

Figure 7 shows the optical image of a water-borne acrylate coating containing the same NIR dye S2161. The dye is more finely dispersed (probably even nearly molecularly distributed in the coating), due to similar solubility of the NIR dye and coating material. The difference in absorption between the dye containing coating (left) and the reference coating (right) is clear. The molecularly distributed dye-containing coating shows a strong absorption, which is detectable with a common digital camera.

The difference in dispersion state can be triggered by the presence of water in the coating and possibly accelerated by acids. This principle is used for the development of a moisture signalling coating. In principle the dissolution process in the coating material can be influenced by the addition of a pH sensitive shell on the particle.

## Claims

1. A coating composition comprising a system comprising a dye encapsulated in a matrix of a polymer, wherein the dye essentially does not absorb in the visible spectrum thereby having no colour for the naked eye while showing absorption in a non-visible part of the spectrum, wherein the visible spectrum is 400-700 nm, and wherein the encapsulated system is able to undergo a detectable change of at least 10% in absorption units (AU) in the absorption properties under the influence of a solvent, preferably moisture.

2. The coating composition according to claim 1, wherein the encapsulated system is in the form of microparticles.

3. The coating composition according to claim 2, wherein the microparticles have a core-shell structure and wherein the core comprises the dye and the shell comprises the polymer.

4. The coating composition according to any one of the preceding claims, wherein the polymer is a pH sensitive polymer having a critical swelling pH, which is preferably under 7 and more preferably in the range 2.5-6.

5. The coating composition according to any one of claims 2-4, wherein the microparticle further comprises a solvent.

6. The coating composition according to claim 5, wherein the dye is dissolved in the solvent, the dye thus being present in a solvated form.

7. The coating composition according to claim 6, wherein the dye is present in the solution in an amount of less than 1 wt.%, preferably less than 0.1 wt.%.

8. The coating composition according to claim 6 or 7, wherein the dye in the solvated form absorbs light with a wavelength in the non-visible part of the light spectrum, and in a non-solvated form it does not absorb light in that part of spectrum.

9. The coating composition according to claim 6 or 7, wherein the dye in the solvated form does not absorb light with a wavelength in the non-visible part of the light spectrum, and in a non-solvated form it absorbs light in that part of spectrum.

10. The coating composition according to any of the preceding claims, wherein the polymer is a pH sensitive polymer which is a copolymer capable of being protonated.

11. The coating composition according to claim 10, wherein the copolymer is polymerised from at least one monomer capable of being protonated, selected from the group consisting of acrylates, methacrylates, vinylics containing at least one primary, secondary or tertiary amine or sulfide group and derivatives thereof.

12. The coating composition according to any one of the preceding claims, wherein the polymer is a pH sensitive polymer which is a hydrolysable polyester, preferably selected from the list consisting of polylactic acid, polyglycolic acid, poly(lactic-co-glycolic acid), polycaprolatones.

13. The coating composition according to any one of the preceding claims, wherein the dye is an IR or a UV dye, and is preferably a non-fluorescent dye.

14. The coating composition according to claim 13, wherein the dye is an IR dye having an absorption peak in the wavelength region 700-2400 nm, preferably 750-1200 nm.

15. The coating composition according to claim 13, wherein the dye is a UV dye having an absorption peak in the wavelength region 10-400 nm.

16. The coating composition according to any one of the preceding claims, wherein the dye is selected from the list consisting of organic dyes, polymeric dyes, metal complexes and plasmonic particles.

17. The coating composition according to claim 16, wherein the dye is an organic dye selected from the list consisting of coumarin, benzophenones, p-aminobenzoic acid, camphor, dibenzoylmethane, homosalate, cinnamates, crylenes and derivatives thereof.

18. The coating composition according to any one of the preceding claims, wherein the encapsulated system further comprises at least one of surfactants, dispersion aids, wetting agents, thickeners, anti-foaming agents.

19. Method to detect moisture in objects, the method comprising the steps of:
(a) providing an object with a signalling layer, said signalling layer comprising the coating composition according to any of claims 1-18, and
(b) measuring the light absorption or reflectance of the coated object in the non-visible part of the light spectrum.

20. The method according to claim 19, wherein the encapsulated system of the dye and the polymer undergoes a change in its light absorption under the influence of moisture.

21. The method according to claim 19 or 20, wherein the step of measuring the light reflectance or absorption is carried out at least twice, the method further comprising the step of detecting differences in the light reflectance or absorption of the object in the non-visible part of the light spectrum.

22. The method according to any one of claims 19-21, wherein the dye changes its state from absorbing to non-absorbing or vice versa, under the influence of a trigger, preferably thermal, mechanical or chemical such as pH, presence of moisture, or corrosion.

23. The method according to any one of claims 19-22, wherein the reflectance of the coated object is detected using a CCD camera, preferably by making at least two images of the coated object, more preferably a first image using a filter and a second image without using the filter, the first and the second image being made at substantially the same point in time or at substantially different points in time.

24. The method according to any one of claims 19-23, wherein the coated surface of the object is a wooden or metal surface, preferably a wooden surface.

25. The method according to claim 24, wherein the wood sort is selected from larch, fir, pine, Douglas fir, maple, birch, beech, ash, meranti, or teak.

26. The coating composition according to claim 1, wherein the system comprises the dye in the matrix of the polymer, wherein the system is in the form of micro particles, wherein the micro particles comprise solid dye particles embedded in the polymer matrix.

## Patentansprüche

1. Beschichtungszusammensetzung, umfassend ein System, umfassend einen in einer Polymermatrix eingekapselten Farbstoff, wobei der Farbstoff im Wesentlichen nicht im sichtbaren Spektrum absorbiert, wodurch er für das bloße Auge keine Farbe hat, während er in einem nicht sichtbaren Teil des Spektrums Absorption zeigt, wobei das sichtbare Spektrum 400 bis 700 nm ist, und wobei das eingekapselte System, unter dem Einfluss eines Lösungsmittels, vorzugsweise Feuchtigkeit, eine nachweisbare Änderung der Absorptionseigenschaften von mindestens 10 % in Absorptionseinheiten (AU) erleben kann.

2. Beschichtungszusammensetzung nach Anspruch 1, wobei das eingekapselte System in Form von Mikroteilchen ist.

3. Beschichtungszusammensetzung nach Anspruch 2, wobei die Mikroteilchen eine Kern-Schale-Struktur haben und wobei der Kern den Farbstoff und die Schale das Polymer umfasst.

4. Beschichtungszusammensetzung nach einem der vorhergehenden Ansprüche, wobei das Polymer ein pH-sensitives Polymer mit einem kritischen Quellungs-pH-Wert ist, der vorzugsweise unter 7 und bevorzugter im Bereich 2,5 bis 6 ist.

5. Beschichtungszusammensetzung nach einem der Ansprüche 2 bis 4, wobei das Mikroteilchen ferner ein Lösungsmittel umfasst.

6. Beschichtungszusammensetzung nach Anspruch 5, wobei der Farbstoff in dem Lösungsmittel gelöst ist, so dass der Farbstoff in solvatisierter Form ist.

7. Beschichtungszusammensetzung nach Anspruch 6, wobei der Farbstoff in der Lösung in einer Menge von weniger als 1 Gew.-%, vorzugsweise weniger als 0,1 Gew.-%, vorhanden ist.

8. Beschichtungszusammensetzung nach Anspruch 6 oder 7, wobei der Farbstoff in der solvatisierten Form Licht mit einer Wellenlänge im nicht sichtbaren Teil des Lichtspektrums absorbiert und er in einer nichtsolvatisierten Form kein Licht in diesem Teil des Spektrums absorbiert.

9. Beschichtungszusammensetzung nach Anspruch 6 oder 7, wobei der Farbstoff in der solvatisierten Form kein Licht mit einer Wellenlänge im nicht sichtbaren Teil des Lichtspektrums absorbiert und er in einer nichtsolvatisierten Form Licht in diesem Teil des Spektrums absorbiert.

10. Beschichtungszusammensetzung nach einem der vorhergehenden Ansprüche, wobei das Polymer ein pH-sensitives Polymer ist, das Copolymer ist, geeignet protoniert zu werden.

11. Beschichtungszusammensetzung nach Anspruch 10, wobei das Copolymer aus mindestens einem Monomer, geeignet protoniert zu werden, polymerisiert ist, ausgewählt aus der Gruppe bestehend aus Acrylaten, Methacrylaten, Vinylverbindungen, enthaltend mindestens eine primäre, sekundäre oder tertiäre Amin- oder Sulfidgruppe und Derivate davon.

12. Beschichtungszusammensetzung nach einem der vorhergehenden Ansprüche, wobei das Polymer ein pH-sensitives Polymer ist, das ein hydrolysierbarer Polyester ist, vorzugsweise ausgewählt aus der Liste bestehend aus Polymilchsäure, Polyglykolsäure, Poly(milchsäure-co-glycolsäure), Polycaprolatonen.

13. Beschichtungszusammensetzung nach einem der vorhergehenden Ansprüche, wobei der Farbstoff ein IR- oder ein UV-Farbstoff ist und vorzugsweise ein nicht fluoreszierender Farbstoff ist.

14. Beschichtungszusammensetzung nach Anspruch 13, wobei der Farbstoff ein IR-Farbstoff mit einem Absorptionspeak im Wellenlängenbereich 700 bis 2400 nm, vorzugsweise 750 bis 1200 nm, ist.

15. Beschichtungszusammensetzung nach Anspruch 13, wobei der Farbstoff ein UV-Farbstoff mit einem Absorptionspeak im Wellenlängenbereich 10 bis 400 nm ist.

16. Beschichtungszusammensetzung nach einem der vorhergehenden Ansprüche, wobei der Farbstoff ausgewählt ist aus der Liste bestehend aus organischen Farbstoffen, polymeren Farbstoffen, Metallkomplexen und plasmonischen Teilchen.

17. Beschichtungszusammensetzung nach Anspruch 16, wobei der Farbstoff ein organischer Farbstoff ist, ausgewählt aus der Liste bestehend aus Cumarin, Benzophenonen, p-Aminobenzoesäure, Kampfer, Dibenzoylmethan, Homosalat, Cinnamaten, Crylenen und Derivaten davon.

18. Beschichtungszusammensetzung nach einem der vorhergehenden Ansprüche, wobei das eingekapselte System ferner mindestens eines von Tensiden, Dispersionshilfsmitteln, Benetzungsmitteln, Verdickungsmitteln, Antischaummitteln umfasst.

19. Verfahren zum Nachweis von Feuchtigkeit in Gegenständen, wobei das Verfahren die Schritte umfasst von:
(a) Versehen eines Gegenstands mit einer Signalisierungsschicht, wobei die Signalisierungsschicht die Beschichtungszusammensetzung nach einem der Ansprüche 1 bis 18 umfasst, und
(b) Messen der Lichtabsorption oder des Reflexionsgrads des beschichteten Gegenstands im nicht sichtbaren Teil des Lichtspektrums.

20. Verfahren nach Anspruch 19, wobei das eingekapselte System aus dem Farbstoff und dem Polymer unter dem Einfluss von Feuchtigkeit eine Änderung seiner Lichtabsorption erlebt.

21. Verfahren nach Anspruch 19 oder 20, wobei der Schritt des Messens der Lichtreflexion oder -absorption mindestens zweimal durchgeführt wird, wobei das Verfahren ferner den Schritt des Nachweisens von Unterschieden in der Lichtreflexion oder -absorption des Gegenstands im nicht sichtbaren Teil des Lichtspektrums umfasst.

22. Verfahren nach einem der Ansprüche 19 bis 21, wobei der Farbstoff seinen Zustand unter dem Einfluss eines vorzugsweise thermischen, mechanischen oder chemischen Auslösers, wie pH, Vorhandensein von Feuchtigkeit oder Korrosion, von absorbierend zu nicht-absorbierend oder umgekehrt ändert.

23. Verfahren nach einem der Ansprüche 19 bis 22, wobei der Reflexionsgrad des beschichteten Gegenstands unter Verwendung einer CCD-Kamera nachgewiesen wird, vorzugsweise durch Aufnahme von mindestens zwei Bildern des beschichteten Gegenstands, bevorzugter eines ersten Bildes unter Verwendung eines Filters und eines zweiten Bildes ohne Verwendung des Filters, wobei das erste und das zweite Bild im Wesentlichen zum gleichen Zeitpunkt oder zu im Wesentlichen unterschiedlichen Zeitpunkten gemacht werden.

24. Verfahren nach einem der Ansprüche 19 bis 23, wobei die beschichtete Oberfläche des Gegenstandes eine Holz- oder Metalloberfläche, vorzugsweise eine Holzoberfläche, ist.

25. Verfahren nach Anspruch 24, wobei die Holzsorte ausgewählt ist aus Lärche, Tanne, Kiefer, Douglasie, Ahorn, Birke, Buche, Esche, Meranti oder Teak.

26. Beschichtungszusammensetzung nach Anspruch 1, wobei das System den Farbstoff in der Matrix des Polymers umfasst, wobei das System in Form von Mikroteilchen ist, wobei die Mikroteilchen feste Farbstoffteilchen umfassen, eingebettet in die Polymermatrix.

## Revendications

1. Composition de revêtement comprenant un système comprenant un colorant encapsulé dans une matrice d'un polymère, dans laquelle le colorant n'absorbe essentiellement pas dans le spectre visible, ne présentant ainsi aucune couleur à l'œil nu tout en montrant des propriétés d'absorption dans une partie non visible du spectre, dans laquelle le spectre visible est de 400 à 700 nm, et dans laquelle le système encapsulé est apte à subir un changement détectable d'au moins 10 % dans les unités d'absorption (AU) des propriétés d'absorption sous l'influence d'un solvant, de préférence de l'humidité.

2. Composition de revêtement selon la revendication 1, dans laquelle le système encapsulé se présente sous la forme de microparticules.

3. Composition de revêtement selon la revendication 2, dans laquelle les microparticules ont une structure cœur-écorce et dans laquelle le cœur comprend le colorant et l'écorce comprend le polymère.

4. Composition de revêtement selon l'une quelconque des revendications précédentes, dans laquelle le polymère est un polymère sensible au pH ayant un pH de gonflement critique, lequel est de préférence inférieur à 7 et plus préférablement dans la plage de 2,5 à 6.

5. Composition de revêtement selon l'une quelconque des revendications 2 à 4, dans laquelle la microparticule comprend en outre un solvant.

6. Composition de revêtement selon la revendication 5, dans laquelle le colorant est dissous dans le solvant, le colorant étant ainsi présent sous une forme solvatée.

7. Composition de revêtement selon la revendication 6, dans laquelle le colorant est présent dans la solution en une quantité inférieure à 1 % en poids, de préférence inférieure à 0,1 % en poids.

8. Composition de revêtement selon la revendication 6 ou 7, dans laquelle le colorant dans la forme solvatée absorbe la lumière avec une longueur d'onde dans la partie non visible du spectre de lumière, et dans une forme non solvatée, il n'absorbe pas la lumière dans cette partie de spectre.

9. Composition de revêtement selon la revendication 6 ou 7, dans laquelle le colorant dans la forme solvatée n'absorbe pas la lumière avec une longueur d'onde dans la partie non visible du spectre de lumière, et dans une forme non solvatée, il absorbe la lumière dans cette partie de spectre.

10. Composition de revêtement selon l'une quelconque des revendications précédentes, dans laquelle le polymère est un polymère sensible au pH qui est un copolymère apte à être protoné.

11. Composition de revêtement selon la revendication 10, dans laquelle le copolymère est polymérisé à partir d'au moins un monomère apte à être protoné, sélectionné dans le groupe consistant en acrylates, méthacrylates, vinyliques contenant au moins une amine primaire, secondaire ou tertiaire ou un groupe sulfure et des dérivés de ceux-ci.

12. Composition de revêtement selon l'une quelconque des revendications précédentes, dans laquelle le polymère est un polymère sensible au pH qui est un polyester hydrolysable, de préférence sélectionné dans la liste consistant en acide polylactique, acide polyglycolique, poly(acide lactique-co-glycolique), polycaprolactones.

13. Composition de revêtement selon l'une quelconque des revendications précédentes, dans laquelle le colorant est un colorant IR ou un colorant UV, et est de préférence un colorant non fluorescent.

14. Composition de revêtement selon la revendication 13, dans laquelle le colorant est un colorant IR présentant un pic d'absorption dans la région de longueur d'onde de 700 à 2400 nm, de préférence de 750 à 1200 nm.

15. Composition de revêtement selon la revendication 13, dans laquelle le colorant est un colorant UV présentant un pic d'absorption dans la région de longueur d'onde de 10 à 400 nm.

16. Composition de revêtement selon l'une quelconque des revendications précédentes, dans laquelle le colorant est sélectionné dans la liste consistant en colorants organiques, colorants polymères, complexes métalliques et particules plasmoniques.

17. Composition de revêtement selon la revendication 16, dans laquelle le colorant est un colorant organique sélectionné dans la liste consistant en coumarine, benzophénones, acide p-aminobenzoïque, camphre, dibenzoylméthane, homosalate, cinnamates, crylènes et dérivés de ceux-ci.

18. Composition de revêtement selon l'une quelconque des revendications précédentes, dans laquelle le système encapsulé comprend en outre au moins l'un parmi tensioactifs, agents de dispersion, agents mouillants, épaississants, agents antimoussants.

19. Procédé pour détecter de l'humidité dans des objets, le procédé comprenant les étapes de :
(a) fourniture d'un objet avec une couche de signalisation, ladite couche de signalisation comprenant la composition de revêtement selon l'une quelconque des revendications 1 à 18, et
(b) mesure de l'absorption de lumière ou de la réflectance de l'objet revêtu dans la partie non visible du spectre de lumière.

20. Procédé selon la revendication 19, dans lequel le système encapsulé du colorant et du polymère subit un changement de son absorption de lumière sous l'influence de l'humidité.

21. Procédé selon la revendication 19 ou 20, dans lequel l'étape de mesure de la réflectance ou de l'absorption de lumière est réalisée au moins deux fois, le procédé comprenant en outre l'étape de détection de différences dans la réflectance ou l'absorption de lumière de l'objet dans la partie non-visible du spectre de lumière.

22. Procédé selon l'une quelconque des revendications 19 à 21, dans lequel le colorant change d'état d'absorbant à non absorbant ou vice versa, sous l'influence d'un déclencheur, de préférence thermique, mécanique ou chimique tel que pH, présence d'humidité ou corrosion.

23. Procédé selon l'une quelconque des revendications 19 à 22, dans lequel la réflectance de l'objet revêtu est détectée à l'aide d'une caméra CCD, de préférence en réalisant au moins deux images de l'objet revêtu, plus préférablement une première image en utilisant un filtre et une deuxième image sans utiliser de filtre, la première et la deuxième image étant réalisées sensiblement au même moment ou à des moments sensiblement différents.

24. Procédé selon l'une quelconque des revendications 19 à 23, dans lequel la surface revêtue de l'objet est une surface en bois ou métallique, de préférence une surface en bois.

25. Procédé selon la revendication 24, dans lequel le type de bois est sélectionné parmi mélèze, sapin, sapin Douglas, érable, bouleau, hêtre, frêne, meranti ou teck.

26. Composition de revêtement selon la revendication 1, dans lequel le système comprend le colorant dans la matrice du polymère, dans laquelle le système se présente sous la forme de microparticules, dans laquelle les microparticules comprennent des particules de colorant solides noyées dans la matrice polymère.
